# EUROPEAN PATENT APPLICATION

(11) **EP 4 782 055 A1**
(43) Date of publication of application: **29.07.2026**
(21) Application number: 26153814.4
(22) Date of filing: 23.01.2026
(51) Int. Cl.: A61N 1/39, A61H 31/00

(54) **SYSTEM OF DEVICES WITH COORDINATED FEEDBACK**

(30) Priority: 23.01.2025 US 202563748934 P; 20.01.2026 US 202619454243
(71) Applicant: STRYKER CORPORATION, Portage, MI 49002-9711 (US)
(72) Inventor: BROSNAN, Daniel Vincent, Portage, 49002 (US); MCLOUTH, Justin, Portage, 49002 (US); SURI, Malini C., Portage, 49002 (US); PATMORE, Kevin M., Portage, 49002 (US)
(74) Representative: V.O.

(57) **Abstract**

An example method includes generating, by a first device, an instruction or an alert; outputting, by the first device, a first output signal indicating the instruction or the alert; transmitting, by the first device to a second device, a communication signal indicating the instruction or the alert; and outputting, by the second device, a second output signal indicating the instruction or the alert. The second output signal has a common characteristic with the first output signal.

## Description

### CROSS-REFERENCE TO RELATED APPLICATION

This application claims priority to U.S. Provisional App. No. 63/748,934, which was filed on January 23, 2025 and is incorporated by reference herein in its entirety.

### BACKGROUND

Various types of medical devices are configured to monitor and treat subjects, such as patients. In medical emergencies, it may be preferrable to utilize multiple medical devices, simultaneously, with the same subject. In addition, multiple providers may be providing care to the same subject. In some cases, it may be beneficial to coordinate efforts in providing care to the subject. However, it can be difficult to coordinate care in rescue scenes involving multiple medical devices and multiple rescuers.

### BRIEF DESCRIPTION OF THE DRAWINGS

FIG. 1 illustrates an example environment for coordinating user feedback by multiple medical devices.
FIG. 2 illustrates example signaling for providing coordinated feedback between different devices within an environment.
FIG. 3 illustrates an example process for providing coordinated feedback.
FIG. 4 illustrates an example of an external defibrillator configured to perform various functions described herein.
FIG. 5 illustrates a chest compression device configured to perform various functions described herein.
FIG. 6 illustrates an example of a monitor of a ventilation device configured to provide coordinated feedback.
FIG. 7 illustrates a ventilation device configured to perform various functions described herein.

### DETAILED DESCRIPTION

Various implementations described herein relate to techniques for providing coordinated feedback from multiple devices. In some examples, implementations of the present disclosure can be utilized by multiple medical devices to provide feedback about treatments, alerts, device conditions, and other relevant information to one or more rescuers. Accordingly, a single rescuer may perceive the coordinated feedback by operating any of a variety of medical devices utilized on a single subject (e.g., patient). Moreover, multiple rescuers can perceive the same coordinated feedback by operating any of the variety of medical devices utilized on the single subject.

In some examples, a first medical device generates an instruction indicating a direction and/or alert related to a subject or to the first medical device itself. In some cases, the direction is to administer a treatment to the subject. In some examples, the alert indicates that a battery level of the first medical device is below a threshold. The first medical device transmits the instruction to one or more second medical devices. In response to receiving the instruction, the second medical device(s) are configured to output signals indicating the direction and/or alert. Simultaneously, the first medical device outputs a signal indicating the direction and/or alert. The signals output by the first medical device and the second medical device(s) may have one or more common characteristics (e.g., frequency, timing, color, tone, duty cycle, intensity, brightness, or any combination thereof), thereby conveying the same direction and/or alert. Accordingly, one or more rescuers operating the first medical device and the second medical device(s) may perceive the same direction and/or alert.

Various examples described herein provide improvements to the technical fields of medical devices and emergency care. In some previous technologies, each medical device utilized on a patient would generate its own directions and/or alerts. Thus, different rescuers operating different medical devices could remain unaware of treatments administered and/or conditions reported by other medical devices utilized on the patient. In some cases, this could result in harm to the patient and the rescuers. For instance, a rescuer manually operating a bag-valve mask (BVM) to provide assisted ventilation to a subject may be unaware that a monitor-defibrillator is preparing to administer an electrotherapy to the same subject, and may therefore be harmed while continuing to touch the BVM when the monitor-defibrillator administers the electrotherapy. In various implementations of this disclosure, such harm can be avoided by causing the BVM itself to warn the rescuer that the monitor-defibrillator is preparing to administer the electrotherapy.

Various implementations of the present disclosure will now be described with reference to the accompanying figures.

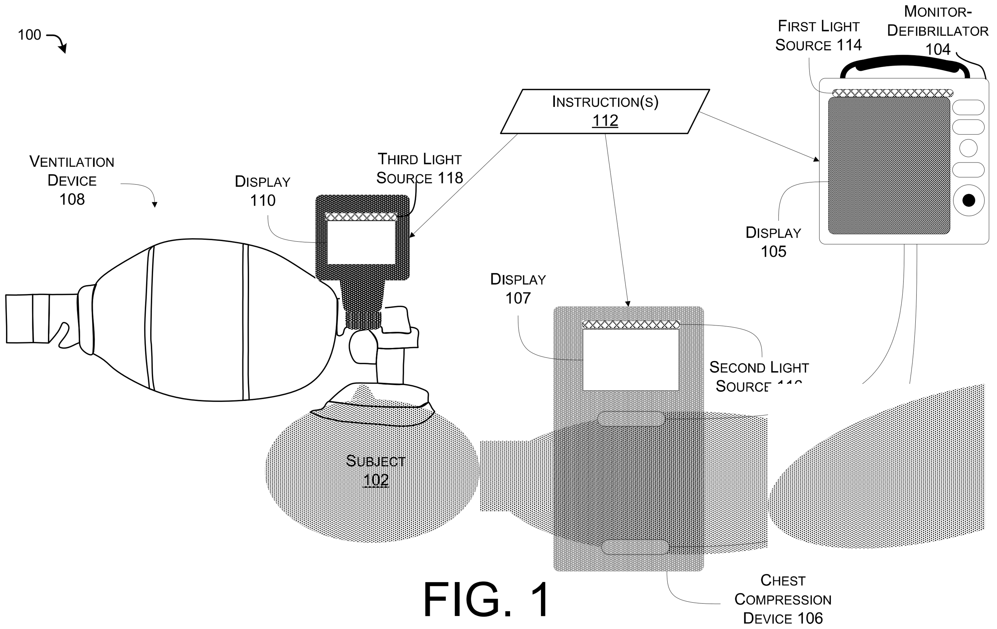

FIG. 1 illustrates an example environment 100 for coordinating user feedback by multiple medical devices. The environment 100 is a rescue scene in which the subject 102 is experiencing an acute medical emergency. In particular cases, the subject 102 lacks spontaneous breathing and/or spontaneous blood circulation. For example, the subject 102 may have an arrhythmia that prevents the heart of the subject 102 from adequately circulating blood through the body of the subject 102. In some cases, the environment 100 is within a clinical environment, such as a hospital, medical clinic, hospice, or the like. In some examples, the environment 100 is in a non-clinical environment, such as a school, office building, airport terminal, or the like.

One or more rescuers (not illustrated) may monitor and/or treat the subject 102 using multiple medical devices. In some cases, one or more of the medical devices are configured to monitor a condition of the subject 102. In some instances, one or more of the medical devices are configured to administer a treatment to the subject 102. One or more of the medical devices are portable devices, for example.

For instance, a monitor-defibrillator 104 is configured to monitor and/or treat the subject 102. In various cases, the monitor-defibrillator 104 is configured to detect one or more physiological parameters of the subject 102. Examples of physiological parameters include an electrocardiogram (ECG), an electrical impedance (e.g., a transthoracic impedance), a blood oxygenation (e.g., a pulse oxygenation (SpO₂), regional oxygenation, cerebral oxygenation, etc.), an airway parameter (e.g., a partial pressure of oxygen (O₂) in the airway of the subject 102, a partial pressure of carbon dioxide (CO₂) in the airway of the subject 102, an airway pressure, a capnograph, an end-tidal airway parameter, such as end-tidal CO₂, a flow rate through the airway of the subject 102, etc.), a blood pressure (e.g., a systolic blood pressure, a diastolic blood pressure, an instantaneous blood pressure in at least one blood vessel, etc.), a blood flow parameter (e.g., a blood velocity, a volumetric flow rate of blood, a pulse wave velocity, etc.), a temperature, an acceleration, or any combination thereof. For instance, the monitor-defibrillator 104 includes, or is communicatively coupled with, one or more sensors, such as electrodes, a blood oxygenation sensor (e.g., pulse oximeter), a gas sensor, a pressure sensor, a flow sensor, a blood pressure cuff, an invasive blood pressure sensor, an ultrasound transducer (e.g., configured to detect blood flow using Doppler-based techniques), a thermometer, an accelerometer, or any combination thereof. The monitor-defibrillator 104 includes a display 105. One or more physiological parameters of the subject 102 may be visually presented on the display 105.

In various examples, the monitor-defibrillator 104 is configured to generate recommendations and/or alerts for the rescuer(s) by analyzing the physiological parameter(s) and/or additional data indicative of the condition of the subject 102. For example, the monitor-defibrillator 104 is configured to detect that the subject 102 has a cardiac arrhythmia by analyzing the ECG of the subject 102. In some cases, the cardiac arrhythmia includes a "shockable arrhythmia," which is a cardiac arrhythmia that is treatable by some type of electrotherapy (e.g., electric shock, pacing pulses, synchronized cardioversion, etc.). For instance, the monitor-defibrillator 104 determines that the ECG is indicative of a condition that may be treatable by administration of an electrical shock to the heart of the subject 102, such as ventricular fibrillation (VF) or pulseless ventricular tachycardia. In some implementations, the monitor-defibrillator 104 determines that the ECG is indicative of bradycardia, which may be treatable by the administration of pacing pulses to the heart of the subject 102. According to some cases, the monitor-defibrillator 104 is further configured to administer the electrotherapy to the subject 102, such as in response to detecting an input signal from a rescuer. In some cases, the display 105 is configured to output an indication of a cardiac condition of the subject 102, an instruction to administer an electrotherapy to the subject 102, or an instruction for rescuers to refrain from touching the subject 102 during the administration of the electrotherapy (e.g., a "hands off" instruction), or any combination thereof. The monitor-defibrillator 104, for instance, administers the electrotherapy by outputting an electrical signal to electrodes disposed on the subject 702. The electrodes, for instance, are disposed on the skin of the chest of the subject 702.

For instance, the monitor-defibrillator 104 is configured to determine that the subject 102 lacks spontaneous circulation by analyzing the blood pressure, blood oxygenation, end-tidal CO₂, ECG, or any combination thereof, of the subject 102. For instance, the monitor-defibrillator 104 infers that the subject 102 lacks spontaneous circulation by determining that the ECG is indicative of a cardiac arrhythmia (such as VF or VT) and/or that another physiological parameter indicative of blood circulation (e.g., blood pressure, blood oxygenation, EtCO₂, or any combination thereof) is below a threshold. In response to determining that the subject 102 lacks spontaneously circulating blood, the monitor-defibrillator 104 may generate an instruction to administer chest compressions to the subject 102. The chest compressions, for instance, may include manual chest compressions. The monitor-defibrillator 104 may output an indication of the physiological parameter(s), whether blood is spontaneously circulating through the body of the subject 102, or one or more instructions to the rescuer(s), on the display 105.

According to some implementations, a chest compression device 106 is configured to administer chest compressions to the subject 102. For instance, a rescuer may place and activate the chest compression device 106 in response to the monitor-defibrillator 104 outputting the instruction to administer the chest compressions to the subject 102. In some cases, the chest compression device 106 includes a compressor (e.g., a plunger or belt) configured to periodically administer pressure to the chest of the subject 102. These compressions on the chest of the subject 102 may force some (e.g., oxygenated) blood through the circulatory system of the body of the subject, thereby preventing hypoxic injury to the body (e.g., the brain and/or vital organs) the subject 102 until the subject 102 regains spontaneous circulation. In some examples, the chest compression device 106 includes a display 107 that communicates information to the rescuer(s). For instance, the display 107 may indicate a current rate, depth, position, or duty cycle of the chest compressions administered to the subject 102. In some cases, the display 107 indicates how long the chest compressions have been administered to the subject 102.

In some examples, the monitor-defibrillator 104 is configured to determine that the subject 102 is not spontaneously breathing by analyzing a capnograph of the subject 102. In some cases, the monitor-defibrillator 104 infers that the subject 102 is not spontaneously breathing in response to determining that the capnograph of the subject 102 lacks local maxima associated with spontaneous exhaling. In response to determining that the subject 102 is not spontaneously breathing, the monitor-defibrillator 104 may generate in instruction to administer assisted ventilation to the subject 102. In some cases, the instruction is output on the display 105 of the monitor-defibrillator 104.

A ventilation device 108 is configured to provide assisted ventilation (e.g., positive pressure ventilation) to the subject 102. For example, a rescuer may place, activate, or otherwise operate the ventilation device 108 in response to the monitor-defibrillator 104 outputting the instruction to administer assisted ventilation to the subject 102. In various cases, the ventilation device 108 includes an airway adaptor that is configured to be fluidically coupled with the airway of the subject 102. Examples of airway adaptors include masks, supraglottic airways, intubation tubes, and the like. The ventilation device 108 also includes a gas source that is fluidically coupled with the airway adaptor and is configured to push a gas into the airway of the subject 102 and/or to draw a gas from the airway of the subject 102. The gas, for instance, includes oxygen and/or air. In some cases, the gas source includes a bag that can push gas into and out of the airway of the subject 102 by being squeezed and released by a rescuer. In some implementations, the gas source includes a mechanical ventilator or other device that automatically administers positive pressure ventilation to the subject 102. In some cases, the ventilation device 108 includes a BVM.

According to some implementations, the ventilation device 108 additionally has monitoring and/or feedback capabilities. For example, the ventilation device 108 may include a monitor including one or more sensors configured to detect parameters within a fluidic circuit that includes the airway of the subject 102, the airway adaptor, the gas source, or any combination thereof. In some cases, the monitor of the ventilation device 108 includes one or more pressure sensors, one or more airflow sensors, one or more gas sensors (e.g., configured to detect a partial pressure of CO₂, oxygen, or some other type of gas within the fluidic circuit), one or more temperature sensors, one or more humidity sensors, or any combination thereof. The monitor of the ventilation device 108, for instance, estimates one or more physiological parameters of the subject 102 based on the parameters detected by the sensor(s). For example, a processor within the monitor of the ventilation device 108 is configured to calculate a tidal volume, respiration rate, or some other derived parameter based on the detected parameter(s). The monitor of the ventilation device 108, in some examples, reports the physiological parameter(s) to the rescuer(s). For example, the ventilation device 108 may include a display 110 configured to indicate the physiological parameter(s) of the subject 102. In some cases, the ventilation device 108 detects and reports whether the subject 102 is spontaneously breathing by analyzing the parameter(s). In some examples, the monitor of the ventilation device 106 is removably coupled with the airway adaptor, the gas source, or one or more tubes connecting the airway adaptor and the gas source. For instance, the monitor of the ventilation device 106 may be reusable on multiple subjects.

In particular cases, the ventilation device 108 is configured to output prompts indicating when to administer ventilation to the subject 102. For example, the ventilation device 108 may analyze the physiological parameter(s) substantially in real-time in order to instruct a rescuer when to squeeze the bag, when to release the bag, or the like. These prompts, for instance, can be output on the display 110.

Each medical device within the environment 100 may have an important, but distinct, role in the care and management of the subject 102. In some cases, multiple rescuers are operating the system of medical devices within the environment 100. For example, one rescuer may be monitoring physiological parameters of the subject 102 output on the display 105 of the monitor-defibrillator 104, one rescuer may be adjusting the parameters of the chest compressions administered by the chest compression device 106, and another rescuer may be manually squeezing and releasing the bag of the ventilation device 108 in order to provide assisted ventilation.

However, in some cases, information identified and output to one rescuer by one medical device may be relevant to the care administered by other rescuers within the environment 100. For example, if the monitor-defibrillator 104 issues a "hands off" instruction because it is about to administer an electrotherapy to the subject 102, it may be beneficial for the rescuer operating the ventilation device 108 to also perceive the instruction in order to avoid being harmed by the electrotherapy being administered through the body of the subject 102 and into the ventilation device 106. In some cases, it may be beneficial for the rescuer operating the monitor-defibrillator 104 to perceive that the chest compression device 106 is administering chest compressions, so that the rescuer may know to refrain from relying on physiological parameters (e.g., blood pressure) that could be significantly distorted from chest compression artifact. If each medical device only visually outputs the instructions, parameters, prompts, or other feedback it generates, then some rescuers may miss important context into the condition and treatment of the subject 102.

It may be possible for a medical device to communicate with a rescuer operating a different medical device using audible signals. For example, the monitor-defibrillator 104 may output an audible instruction to refrain from touching the subject 102 before the monitor-defibrillator 104 outputs an electrotherapy to the subject 102, and the rescuer operating the ventilation device 108 may hear the audible instruction without looking away from the display 110 of the ventilation device 108. However, audible feedback has some drawbacks. In a high-stress rescue scene, it may be difficult for rescuers to concentrate on their individual tasks when they are perceiving audible prompts from multiple medical devices. Moreover, if two medical devices output audible prompts simultaneously, it may be difficult for rescuers to perceive both prompts.

According to various implementations of the present disclosure, these and other problems can be addressed by coordinating feedback from the medical devices in the environment 100. In various cases, the monitor-defibrillator 104, the chest compression device 106, the ventilation device 108, or any combination thereof, are configured to generate and transmit at least one instruction 112. The monitor-defibrillator 104, the chest compression device 106, the ventilation device 108, or any combination thereof, are configured to receive the instruction(s) 112. In various cases, the medical devices within the environment 100 are configured to output prompts, instructions, or other types of feedback based on the received instruction(s) 112.

The instruction(s) 112 are transmitted over one or more communication interfaces. For example, at least a portion of the instruction(s) 112 may be transmitted between transceivers over one or more wireless interfaces, such as near-field communication (NFC) interfaces, BLUETOOTH^{™} interfaces, cellular interfaces (e.g., a 3GPP interface), WI-FI interfaces, any other type of communication interface described herein, or any combination thereof. In some cases, at least a portion of the instruction(s) 112 is transmitted between ports coupled to one or more wired interfaces, such as electrical cables, optical cables, or any combination thereof. The monitor-defibrillator 104, the chest compression device 106, and the ventilation device 108 are communicatively coupled with one another. In some cases, the medical devices within the environment 100 are nodes within a mesh network. In some cases, one of the medical devices acts as a "server" device and the other medical devices serves as a "client" device within the environment 100. In some cases, the instruction(s) 112 are transmitted and received in continuous streams of data packets transmitted between the medical devices in the environment 100.

In various implementations, the instruction(s) 112 include directions to output a user prompt to administer a treatment to the subject 102 and/or to pause the treatment. For example, the instruction(s) 112 may include directions to administer assisted ventilation to the subject 102, to administer chest compressions to the subject 102, to adjust a treatment parameter (e.g., a compression parameter, a ventilation parameter, or the like), to administer an electrotherapy to the subject 102, to administer a medication to the subject 102, to pause any treatment described herein, or any combination thereof. In some implementations, the treatment is periodic. For example, the instruction(s) 112 may include directions to administer the treatment at predetermined intervals, at predetermined times, or at a predetermined frequency. In some cases, the instruction(s) 112 include timing information indicating the time at which the treatment is to be administered to the subject 102. In particular cases, the instruction(s) 112 indicate a direction to administer a dose of a paralytic (e.g., succinylcholine, rocuronium, or the like) and/or a sedative (e.g., etomidate, propofol, ketamine, fentanyl, or the like) to the subject 102, such as in a situation in which a paralytic and/or sedative used to intubate the subject 102 has begun to wear off. In some cases, the wearing off of the paralytic and/or sedative can be detected by detecting an artifact in an airway parameter of the subject 102 indicating that the subject 102 has some spontaneous respiratory activity. In some examples, the wearing off of the paralytic and/or sedative is predicted based on a time that has expired since the subject 102 last received a dose of the paralytic and/or sedative (e.g., as input into one of the medical devices by a user).

According to some cases, the instruction(s) 112 include directions to measure a physiological parameter of the subject 102. For instance, the instruction(s) 112 may include directions to activate a sensor, to place a sensor, or the like. According to some cases, the instruction(s) 112 indicate an alert based on a determination that one or more physiological parameters of the subject 102 are outside of a threshold range.

In some examples, the instruction(s) 112 include directions to perform another task within the environment 100. For instance, the instruction(s) may include directions to refrain from touching the subject 102 (e.g., a "hands off" message in advance of administration of an electrotherapy to the subject 102), to transport the subject 102 to a clinical environment, or the like. In some cases, the instruction(s) 112 include an indication of a condition of one or more of the medical devices in the environment 100. For instance, the instruction(s) 112 may indicate that a battery level of one of the medical devices is below a threshold, that one of the medical devices is malfunctioning, or that a consumable component of one of the medical devices (e.g., a disposable sensor, electrode, or the like) has been expired.

According to some examples, the instruction(s) 112 include directions for more than one rescuer in the environment 100. Upon receiving the instruction(s) 112, the various medical devices within the environment 100 are configured to output coordinated signals to the multiple rescuers that indicate the directions indicated by the instruction(s) 112. In particular cases, the coordinated signals include visual signals, rather than audible signals. In some cases, the light source of the medical device transmitting the instruction(s) 112 also outputs coordinated signals indicating the directions indicated in the instruction(s) 112.

In various cases, the medical devices include light sources configured to output the coordinated signals to the rescuers. For instance, the monitor-defibrillator 104 includes a first light source 114, the chest compression device 106 includes a second light source 116, and the ventilation device 108 includes a third light source 118. One or more of the light sources include bar-shaped lights. In some cases, the light sources include one or more light-emitting diodes (LEDs) configured to emit light at different pulse patterns, brightnesses, colors, frequencies, and duty cycles. In various cases, a circuit including an example light source is configured to supply an electrical signal to the light source based on the instruction(s) 112. One or more of the light sources, for instance, are disposed adjacent to one or more user interfaces of the medical devices. In some cases, a light source is disposed at an edge of a display of a medical device, or at an edge of a control panel (e.g., including one or more buttons or indicators) of the medical device, or disposed on a side of a housing of the medical device that is adjacent to one or more user interfaces of the medical device. For example, the first light source 114 is disposed at an edge of the display 105, the second light source 116 is disposed at an edge of the display 107, and the third light source 118 is disposed at an edge of the display 110. In some instances, the display 107, for instance, is substituted for a panel including one or more user interface elements, such as buttons, status indicators (e.g., light sources indicating power or operation status), dials, and the like. The second light source 116, for instance, may be disposed at an edge of the panel of the chest compression device 106.

In various cases, one or more of the light sources are disposed on one or more sides of the respective housings of the medical device(s). Thes side(s), for instance, are adjacent to sides of the housing on which the respective displays (or user interface panels) are disposed. For example, the first light source 114 is disposed on one or more sides of the housing of the monitor-defibrillator 104 that extend from a side of the housing on which the display 105 is disposed; the second light source 116 is disposed on one or more sides of the housing of the chest compression device 106 on which the display 107 (or panel) is disposed; the third light source 118 is disposed on one or more sides of the housing of the monitor of the ventilation device 108 that extend from a side of the housing on which the display 110 is disposed; or any combination thereof. In some cases, an example light source is disposed on a side that is nonparallel to the side on which its corresponding display (or panel) is disposed. Accordingly, the light source may be visible from different positions in the environment 100 (or at different angles) than its corresponding display (or panel). For example, if the housing of the monitor of the ventilation device 108 has a substantially cubic shape, and the display 110 is disposed on a first side of the cubic shape, the third light source 118 may be disposed on one to four of the sides of the cubic shape that extend from the first side. In various cases, the display 110 is configured to output a visual single in a first direction, and the third light source 118 is configured to output a signal in one or more second directions that are nonparallel to the first direction (e.g., 90° with respect to the first direction along one or more planes).

In various cases, coordinated signals output by the light sources can be readily perceived by rescuers viewing the various displays of the medical devices in the environment 100. In some examples, a coordinated signal output by one of the light sources can be perceived from a different direction than the display disposed adjacent to the light source. For example, a first rescuer may be positioned in a first position in the environment 100 at which the first rescuer can view the display 105 of the monitor-defibrillator 104. The display 105, however, is not visible to a second rescuer positioned in a second position in the environment 100. In various cases, the first light source 114 emits a coordinated signal toward the second position, such that the second rescuer may perceive the coordinated signal without necessarily viewing the display 105. Various factors can impact the directionality of the coordinated signal output by the light sources. For example, the direction of a coordinated signal may be impacted by the location of the light source on its respective medical device (e.g., what side of the housing the light source is disposed), one or more lenses integrated with the light source, one or more mirrors integrated with the light source, or other elements integrated with the light source that are configured to refract and/or scatter light emitted by the light source in one or more directions.

In some examples, a common characteristic of the coordinated signals is associated with a type of direction output to the rescuers. For instance, the medical devices may simultaneously output light signals having the same color, brightness, and pulse pattern to indicate a coordinated direction to the rescuers within the environment 100. In various cases, the light sources may be configured to illuminate in different colors to indicate different types of directions, such as directions to administer different types of treatments, to detect different types of parameters, or to perform other types of actions. In some cases, one color may signify a direction to administer assisted ventilation to the subject 102, another color may signify a direction to administer chest compressions to the subject 102, and yet another color may signify a direction to refrain from touching the subject 102 (e.g., during an electrotherapy administered to the subject 102). In some examples, one color is associated with one phase of the treatment (e.g., compressing the chest of the subject 102) and another color is associated with another phrase of the treatment (e.g., releasing the chest of the subject 102).

In some cases, the coordinated signals have different pulse patterns signifying different directions to one or more rescuers. For instance, in some cases, the light sources blink at a first pulse pattern to indicate a first type of direction, and blink at a second pulse pattern to indicate a different type of direction. In some cases, the pulse pattern of the coordinated signal indicates the timing of the action directed by the instruction(s) 112. For example, the light sources may illuminate when prompting the rescuers to squeeze the ventilation device 108 to provide assisted ventilation. In some instances, the light sources illuminate when prompting the rescuers to compress the chest of the subject 102 or to release the chest of the subject 102. In some cases, the light sources illuminate, or issue a particular pulse pattern, when warning the rescuers to refrain from touching the subject 102 during administration of an electrotherapy. In some examples, the instruction(s) 112 indicate a timing of the pulse patterns (and the directions), a frequency of the pulse patterns, a duty cycle of the pulse patterns, or any combination thereof. The timing of the pulse patterns, for instance, corresponds to the timing at which the rescuers are directed to perform one or more actions.

According to some cases, the coordinated signals output by the light sources are configured to adjust based on a real-time condition of the subject 102. In some cases, the coordinated signals are adjusted to reflect an alteration of an action directed to the rescuers within the environment 100. For example, the instruction(s) 112 transmitted by the monitor-defibrillator 104 may initially instruct the chest compression device 106 and the ventilation device 108 to output coordinated signals that direct rescuers to provide assisted ventilation to the subject 102 at a first frequency and/or duty cycle. However, the monitor-defibrillator 104 may later predict, by detecting that a blood oxygenation level of the subject 102 is below a threshold, that the condition of the subject 102 will be improved if the subject 102 receives ventilation at second frequency and/or duty cycle. In response, the monitor-defibrillator 104 may output the instruction(s) 112 to direct the chest compression device 106 and the ventilation device 108 to output coordinated signals that direct the rescuers to provide assisted ventilation to the subject 102 at the second frequency and/or duty cycle. In some cases, a first instruction among the instruction(s) 112 is an instruction to activate a coordinated signal (e.g., a direction to administer chest compressions to the subject 102) and a subsequent second instruction among the instruction(s) 112 is an instruction to deactivate the coordinated signal (e.g., cease outputting the direction to administer the chest compressions to the subject 102).

In some cases, the instruction(s) 112 cause one or more of the medical devices to output the coordinated signals at the same time in an on-demand and/or scheduled fashion. In some respects, a primary medical device (e.g., the monitor-defibrillator 104) outputs the instruction(s) 112 to secondary medical devices (e.g., the chest compression device 106 and the ventilation device 108) that cause the secondary medical devices to output coordinated signals at the same time in response to receiving the instruction(s) 112. In some cases, the medical devices are preconfigured to operate at a coordinated time scale. For instance, respective clocks of the medical devices can be synchronized, such as by exchanging communications that cause the secondary medical devices to synchronize their clocks to the primary medical device, by communicating with an external server (e.g., utilizing the Berkeley algorithm), in a distributed fashion using network time protocol (NTP)-based techniques, based on receiving the same broadcast signal from a satellite (e.g., a global positioning system (GPS) satellite), or any combination thereof. Once the medical devices are operating on the same time scale, the medical devices may output coordinated signals at simultaneous, prescheduled times indicated in the instruction(s) 112.

Although FIG. 1 illustrates examples of visual coordinated feedback, implementations are not so limited. For example, the instruction(s) 112, in some cases, cause the medical devices to simultaneously output audible signals, or to vibrate, with one or more common characteristics (e.g., a common timing, a common wavelength, a common pattern, or a common intensity) indicating the same direction.

According to various implementations, the coordinated signals output by the light sources of the medical devices can indicate device conditions, in addition to or instead of conditions associated with the physiological state of the subject 102. For example, the timing, frequency, color, pulse pattern, brightness, or other feature of the coordinated signals may indicate a condition of the monitor-defibrillator 104, the chest compression device 106, the ventilation device 108, or any combination thereof. Examples of device conditions include a charge level of a battery of a device (e.g., whether the charge level is below a predetermined threshold), a malfunction associated with the device, an expired consumable component of the device (e.g., whether electrodes connected to the monitor-defibrillator 104 are expired), detected movement of the device (e.g., movement that could increase artifact in a parameter detected by the device and/or interfere with a treatment output by the device), communicative coupling of the device, or any combination thereof. In some cases, one medical device indicates its own device condition to the other medical devices in the instruction(s) 112, thereby causing at least one of the other medical devices to output coordinated feedback indicating the device condition. In some examples, a first medical device detects the device condition of a second medical device, and communicates the device condition to a third medical device in the instruction(s) 112. For instance, the monitor-defibrillator 104 may detect that the ventilation device 108 is no longer communicatively coupled with the monitor-defibrillator 104 (e.g., by attempting to ping the ventilation device 108), and may cause the chest compression device 106 to output coordinated feedback by transmitting the instruction(s) 112 to the chest compression device 106 indicating that communication with the ventilation device 108 has been interrupted.

A particular example will now be described with reference to FIG. 1. In this example, the monitor-defibrillator 104 detects a blood oxygenation and EtCO₂ of the subject 102. The monitor-defibrillator 104 determines that the subject 102 has ceased spontaneous breathing by determining that the blood oxygenation and EtCO₂ of the subject 102 have deteriorated. In response to determining that the subject 102 has ceased spontaneously breathing, the monitor-defibrillator 104 outputs the instruction(s) 112 indicating a direction to administer assisted ventilation to the subject 102. For instance, the direction may indicate predetermined timing of prompts to apply assisted ventilation to the subject 102, such as a predetermined timing associated with providing ten ventilation cycles to the subject 102 per minute.

In response to receiving the instruction(s) 112, the chest compression device 106 and the ventilation device 108 output coordinated signals indicating the direction to administer assisted ventilation to the subject 102 at the predetermined timing. In addition, the monitor-defibrillator 104 outputs coordinated signals indicating the same direction. For example, the first light source 114, the second light source 116, and the third light source 118 simultaneously output ten pulses of blue light (e.g., indicating the ventilation direction) per minute.

In various cases, the ventilation device 108 is configured to detect a partial pressure of CO₂ within a fluid circuit including the airway of the subject 102, as well as the airway adapter and gas source of the ventilation device 108. The ventilation device 108 may determine, by analyzing the partial pressure of CO₂ within the fluid circuit, that the subject 102 is being hyperventilated by determining that the EtCO₂ of the subject 102 is lower than a threshold.

In response to detecting hyperventilation of the subject 102, the ventilation device 108 outputs the instruction(s) 112 indicating a direction to output an alert indicating hyperventilation. For example, upon receiving the instruction(s) 112 from the ventilation device 108, the monitor-defibrillator 104 and the chest compression device 106 output coordinated signals indicating the alert. In some examples, the first light source 114, the second light source 116, and the third light source 118 simultaneously output a red light signal indicating the hyperventilation.

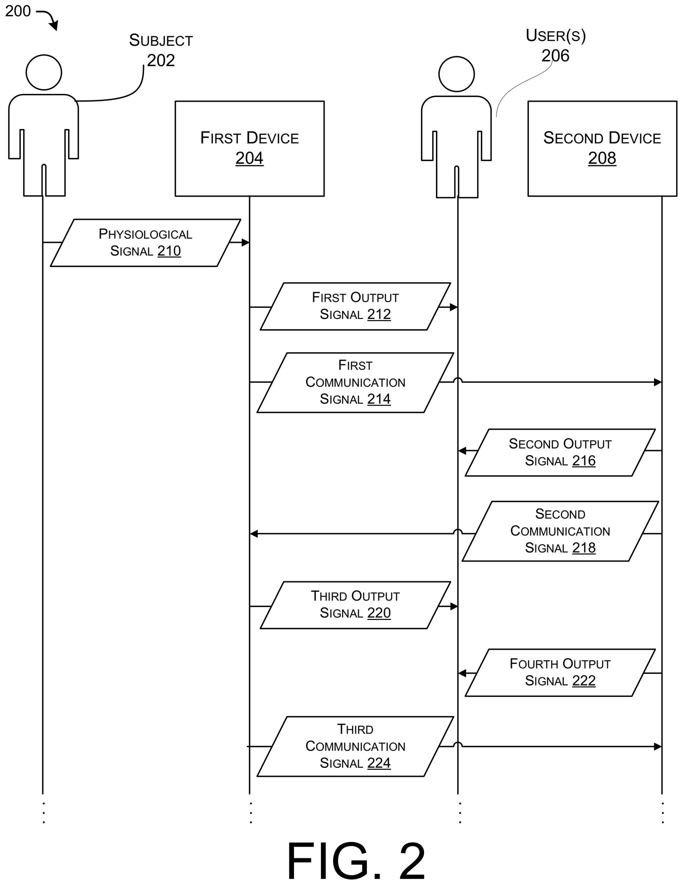

FIG. 2 illustrates example signaling 200 for providing coordinated feedback between different devices within an environment. For instance, the signaling 200 is between a subject 202 (e.g., the subject 102), a first device 204 (e.g., a computing device, the monitor-defibrillator 104, the chest compression device 106, or the ventilation device 108), at least one user 206 (e.g., at least one rescuer), and a second device 208 (e.g., a computing device, the monitor-defibrillator 104, the chest compression device 106, or the ventilation device 108). Various signals illustrated in FIG. 2 are transmitted over one or more wired interfaces and/or one or more wireless interfaces. At least some of the signals illustrated in FIG. 2 may be encoded with various types of data in accordance with one or more communication protocols.

The first device 204 is configured to detect a physiological signal 210 from the subject 202. For example, the physiological signal 210 may include an electrical signal, a pressure, a force, an acceleration, a temperature, a light transmittance, a touch, a sound, or any combination thereof. In some cases, the first device 204 includes, or is communicatively coupled with, a sensor configured to detect the physiological signal 210. In some examples, the first device 204 detects a physiological parameter of the subject 202 based on the physiological signal 210. For instance, the first device 204 may determine that the subject 202 has ceased spontaneously breathing by determining that the transthoracic impedance of the subject 202 lacks peaks consistent with spontaneous breathing. Optionally, the first device 204 determines the physiological parameter, or performs an analysis of the condition of the subject 202, based on the physiological signal 210 and another signal received from an external medical device (not illustrated). For example, the first device 204 may detect a blood pressure of the subject 202 based on the physiological signal 210, may also receive an indication of an EtCO₂ of the subject 202 based on a communication from a separate medical device, and may infer that the subject 202 has a return of spontaneous circulation by analyzing both the blood pressure and the EtCO2 of the subject 202. According to various implementations, the first device 204 generates a direction, instruction, alert, or other communication based on the physiological parameter and/or condition of the subject 202.

The first device 204 is configured to provide a first output signal 212 to the user(s) 206. In various cases, the first output signal 212 indicates the direction, instruction, alert, or other communication. For instance, the first output signal 212 indicates a direction to perform one or more actions. For example, the first output signal 212 may direct the user(s) 206 to administer a treatment, such as assisted ventilation, on the subject 202. In various cases, the first output signal 212 includes a visual signal. For instance, the first output signal 212 is a light signal output by a light source integrated with the first device 204. In some cases, the light source is a light bar disposed on an edge of a display of the first device 204. In some examples, a color, brightness, and/or pulse pattern of the first output signal 212 is indicative of the action that the first device 204 is instructing the user(s) 206 to perform. For example, the color of the first output signal 212 is indicative of a direction to apply assisted ventilation to the subject 202. Moreover, the pulse pattern of the first output signal 212 may indicate a timing at which the first device 204 is instructing the assisted ventilation to be performed on the subject 202.

The first device 204 is further configured to provide a first communication signal 214 to the second device 208. In various cases, the first communication signal 214 indicates the direction, instruction, alert, or other communication indicated by the first output signal 212. For instance, the first communication signal 214 also indicates the direction to administer the treatment (e.g., assisted ventilation) to the subject 202. In some examples, the first communication signal 214 encodes the type of action to be directed, the frequency of the action, the timing of the action, or any combination thereof.

In response to receiving the first communication signal 214, the second device 208 is configured to provide a second output signal 216 to the user(s) 206. The second output signal 216 includes a direction to perform the one or more actions directed by the first output signal 212. For example, the second output signal 216 may direct the user(s) 206 to administer the treatment, such as assisted ventilation, on the subject 202. In various cases, the second output signal 216 includes a visual signal. For instance, the second output signal 216 is a light signal output by a light source integrated with the second device 208. In some cases, the light source is a light bar disposed on an edge of a display of the second device 208. In some examples, a color, brightness, and/or pulse pattern of the second output signal 216 is indicative of the action that the second device 208 is instructing the user(s) 206 to perform. For example, the color of the second output signal 216 is indicative of the direction to apply assisted ventilation to the subject 202. Moreover, the pulse pattern of the second output signal 216 may indicate a timing at which the second device 208 is instructing the assisted ventilation to be performed on the subject 202.

The second output signal 216 is consistent with the first output signal 212, in various implementations. For example, the first output signal 212 and the second output signal 216 may have the same color, brightness, and/or pulse pattern, despite being output by different devices. Moreover, in some cases, the first output signal 212 is perceived by a first user among the user(s) 206, and the second output signal 216 is perceived by a second user among the user(s) 206. Accordingly, both the first user and the second user may be made aware of the direction.

The second device 208 is further configured to provide a second communication signal 218 to the first device 204. In some cases, the second device 208 generates a direction to perform a different action. For example, the second device 208 may determine (e.g., by comparing a blood oxygenation of the subject 202 to a threshold and/or determining that the blood oxygenation is lower than the threshold) that chest compressions should be administered to the subject 202. In various cases, the second communication signal 218 indicates the direction to perform the different action. For example, the second communication signal 218 indicates the direction to administer chest compressions to the subject 202. The second communication signal 218, for instance, indicates the different action, a frequency of the different action, a timing of the different action, or any combination thereof.

In response to receiving the second communication signal 218, the first device 204 is configured to provide a third output signal 220 to the user(s) 206. The third output signal 220 includes the direction to perform the different action. For example, the third output signal 220 may direct the user(s) 206 to administer the different action, such as chest compressions, on the subject 202. In various cases, the third output signal 220 includes a visual signal. For instance, the third output signal 220 is a light signal output by the light source integrated with the first device 204. In some cases, the light source is the light bar disposed on the edge of the display of the first device 204. In some examples, a color and/or pulse pattern of the third output signal 220 is indicative of the action that the first device 204 is instructing the user(s) 206 to perform. For example, the color of the third output signal 220 is indicative of the direction to apply chest compressions to the subject 202. Moreover, the pulse pattern of the third output signal 220 may indicate a timing at which the first device 204 is instructing the chest compressions to be performed on the subject 202. In some cases, the first device 204 refrains from outputting the first output signal 212 when the first device 204 begins outputting the third output signal 220.

The second device 208 is configured to provide a fourth output signal 222 to the user(s) 206. The fourth output signal 222 includes the direction to perform the different action. For example, the fourth output signal 222 may direct the user(s) 206 to administer the different action, such as chest compressions, on the subject 202. In various cases, the fourth output signal 222 includes a visual signal. For instance, the fourth output signal 222 is a light signal output by the light source integrated with the second device 208. In some cases, the light source is the light bar disposed on the edge of the display of the second device 208. In some examples, a color and/or pulse pattern of the fourth output signal 222 is indicative of the action that the second device 208 is instructing the user(s) 206 to perform. For example, the color of the fourth output signal 222 is indicative of the direction to apply chest compressions to the subject 202. Moreover, the pulse pattern of the fourth output signal 222 may indicate a timing at which the second device 208 is instructing the chest compressions to be performed on the subject 202. In some cases, the second device 208 refrains from outputting the second output signal 216 when the second device 208 begins outputting the fourth output signal 222.

The third output signal 220 is consistent with the fourth output signal 222, in various implementations. For example, third output signal 220 and the fourth output signal 222 may have the same color and/or pulse pattern, despite being output by different devices. Moreover, in some cases, the third output signal 220 is perceived by the second user among the user(s) 206, and the fourth output signal 222 is perceived by the first user among the user(s) 206. Accordingly, both the first user and the second user may be made aware of the new direction.

In some cases, the first device 204 determines to pause the treatment directed by the third output signal 220 and the fourth output signal 222. For instance, the first device 204 may determine that the subject 202 has a return of spontaneous circulation (ROSC), such that the blood of the subject 202 can effectively circulate throughout the body of the subject 202 without the subject 202 receiving chest compressions. The first device 204 may refrain from outputting the third output signal 220 in response to determining to pause the treatment. The first device 204 is additionally configured to output a third communication signal 224 to the second device 208. The third communication signal 224, in various cases, indicates a direction to pause the treatment. In response to receiving the third communication signal 224, in various examples, the second device refrains from outputting the fourth output signal 222 to the user(s) 206.

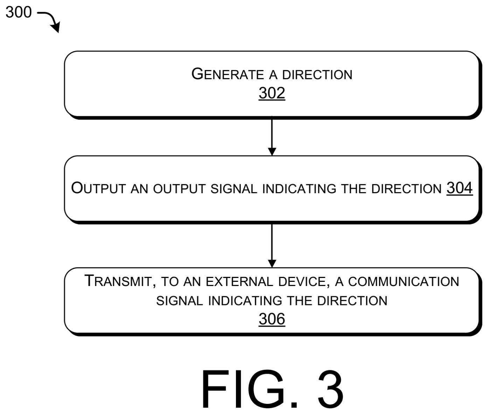

FIG. 3 illustrates an example process 300 for providing coordinated feedback. The process 300 is performed by an entity, such as a computing device, at least one processor, a medical device, an automated external defibrillator (AED), the monitor-defibrillator 104, the chest compression device 106, the ventilation device 108, the first device 204, the second device 208, or any combination thereof.

At 302, the entity generates a direction. In some cases, the direction includes an instruction to perform an action. For instance, the action may be to administer a treatment (e.g., assisted ventilation, chest compressions, an electrotherapy, medication administration, or the like) to a subject. In some examples, the direction includes an alert. For instance, the alert may indicate that a condition of the subject is deteriorating. In some cases, the alert indicates that a treatment is about to be performed on the subject that could harm a user touching the subject. According to some examples, the alert is a device alert. For instance, the alert may indicate that a battery level of a device (e.g., the entity) is below a threshold charge level, that a consumable accessory (e.g., defibrillation electrodes, a disposable element, or the like) of the device has expired, or any combination thereof.

In some implementations, the entity generates the direction by analyzing one or more physiological parameters of the subject. For example, the entity determines that the subject has a condition by analyzing the physiological parameter(s). The entity may detect the physiological parameter. In some cases, the entity receives an indication of the physiological parameter from an external device that is communicatively coupled with the entity. In cases in which the entity identifies multiple estimates of the physiological parameter (e.g., an estimate detected by the entity itself and an estimate detected by the external device), the entity may select one estimate as a prioritized estimate, or analyze a combination of the estimates. In some cases, the selection of the prioritized estimate may be based on a predetermined rule, such as a rule indicating that the estimate by the external device should be prioritized over the estimate by the entity, or vice versa. Examples of conditions include spontaneous circulation, lack of spontaneous circulation, spontaneous respiration, lack of spontaneous respiration, ineffectiveness of a paralytic, a cardiac arrhythmia, or resolution of a cardiac arrhythmia. In some cases, the entity determines that the condition of the subject has deteriorated. For example, the entity may determine that a physiological parameter of the subject has fallen below a threshold, a first derivative of the physiological parameter with respect to time is below a threshold, or a combination thereof.

At 304, the entity outputs an output signal indicating the direction. The entity, in various implementations, includes an output device that provides the output signal to a user. For example, the output device includes at least one light source. In various cases, the output device includes a bar-shaped light source disposed on an edge of a display of the entity. The display may be separate than the output device. In various cases, the output signal includes a light signal. For instance, a color, pulse pattern, or other characteristic of the light signal indicates the direction, instruction, alert, or any combination thereof. In some cases, the color, pulse pattern, or other characteristic of the light signal further indicates a timing at which an action (e.g., a treatment administered to the subject) is instructed to occur.

At 306, the entity transmits, to an external device, a communication signal indicating the direction. In various implementations, the communication signal causes the external device to output an additional output signal indicating the direction. For example, the external device also includes an output device (e.g., a bar-shaped light source disposed on an edge of a display of the external device) configured to output a light signal indicating the direction. In various implementations, the external device outputs the additional output signal simultaneously as the entity is outputting the other output signal. For example, one or more characteristics of the output signals are consistent. For instance, the additional output signal has the same color, pulse pattern, or other characteristic indicating the direction, instruction, alert, or any combination thereof. In some cases, the communication signal indicates a frequency, duty cycle, or other type of timing characteristic. Accordingly, the output signal output by the external device may be emitted by the external device simultaneously with the output signal output by the entity itself.

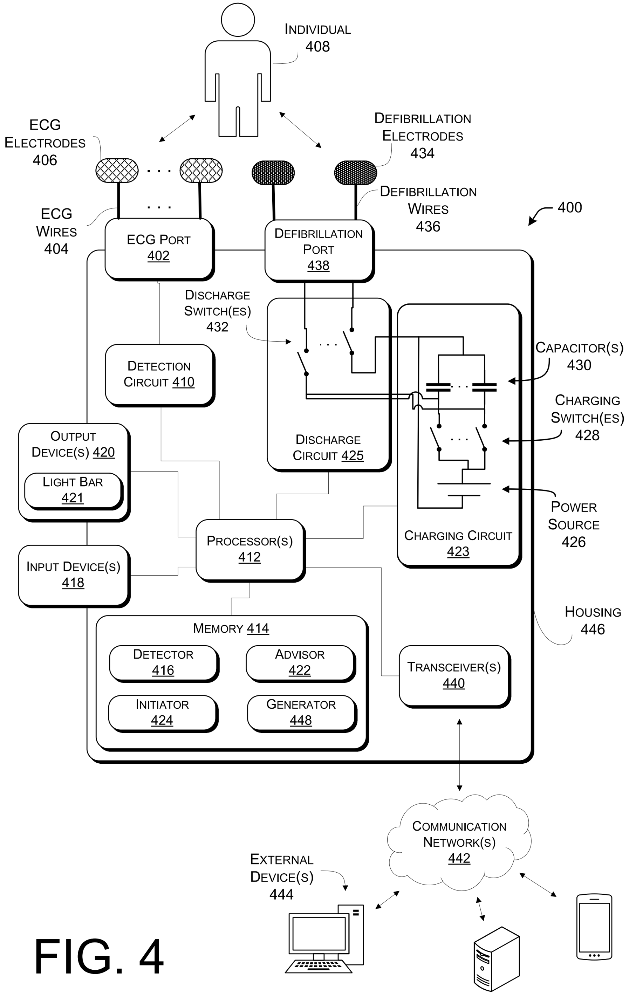

FIG. 4 illustrates an example of an external defibrillator 400 configured to perform various functions described herein. For example, the external defibrillator 400 is the monitor-defibrillator 104 described above with reference to FIG. 1.

The external defibrillator 400 includes an electrocardiogram (ECG) port 402 connected to multiple ECG wires 404. In some cases, the ECG wires 404 are removeable from the ECG port 402. For instance, the ECG wires 404 are plugged into the ECG port 402 via connectors. The ECG wires 404 are connected to ECG electrodes 406, respectively. In various implementations, the ECG electrodes 406 are disposed on different locations on an individual 408. A detection circuit 410 is configured to detect relative voltages between the ECG electrodes 406. These voltages are indicative of the electrical activity of the heart of the individual 408.

In various implementations, the ECG electrodes 406 are in contact with the different locations on the skin of the individual 408. In some examples, a first one of the ECG electrodes 406 is placed on the skin between the heart and right arm of the individual 408, a second one of the ECG electrodes 406 is placed on the skin between the heart and left arm of the individual 408, and a third one of the ECG electrodes 406 is placed on the skin between the heart and a leg (either the left leg or the right leg) of the individual 408. In these examples, the detection circuit 410 is configured to measure the relative voltages between the first, second, and third ECG electrodes 406. Respective pairings of the ECG electrodes 406 are referred to as "leads," and the voltages between the pairs of ECG electrodes 406 are known as "lead voltages." In some examples, more than three ECG electrodes 406 are included, such that 5-lead or 12-lead ECG signals are detected by the detection circuit 410.

The detection circuit 410 includes at least one analog circuit, at least one digital circuit, or a combination thereof. The detection circuit 410 receives the analog electrical signals from the ECG electrodes 406, via the ECG port 402 and the ECG wires 404. In some cases, the detection circuit 410 includes one or more analog filters configured to filter noise and/or artifact from the electrical signals. The detection circuit 410 includes an analog-to-digital (ADC) in various examples. The detection circuit 410 generates a digital signal indicative of the analog electrical signals from the ECG electrodes 406. This digital signal can be referred to as an "ECG signal" or an "ECG."

In some cases, the detection circuit 410 further detects an electrical impedance between at least one pair of the ECG electrodes 406. For example, the detection circuit 410 includes, or otherwise controls, a power source that applies a known voltage (or current) across a pair of the ECG electrodes 406 and detects a resultant current (or voltage) between the pair of the ECG electrodes 406. The impedance is generated based on the applied signal (voltage or current) and the resultant signal (current or voltage). In various cases, the impedance corresponds to respiration of the individual 408, chest compressions performed on the individual 408, and other physiological states of the individual 408. In various examples, the detection circuit 410 includes one or more analog filters configured to filter noise and/or artifact from the resultant signal. The detection circuit 410 generates a digital signal indicative of the impedance using an ADC. This digital signal can be referred to as an "impedance signal" or an "impedance."

The detection circuit 410 provides the ECG signal and/or the impedance signal one or more processors 412 in the external defibrillator 400. In some implementations, the processor(s) 412 includes a central processing unit (CPU), a graphics processing unit (GPU), both CPU and GPU, or other processing unit or component known in the art.

The processor(s) 412 is operably connected to memory 414. In various implementations, the memory 414 is volatile (such as random access memory (RAM)), non-volatile (such as read only memory (ROM), flash memory, etc.) or some combination of the two. The memory 414 stores instructions that, when executed by the processor(s) 412, causes the processor(s) 412 to perform various operations. In various examples, the memory 414 stores methods, threads, processes, applications, objects, modules, any other sort of executable instruction, or a combination thereof. In some cases, the memory 414 stores files, databases, or a combination thereof. In some examples, the memory 414 includes, but is not limited to, RAM, ROM, electrically erasable programmable read-only memory (EEPROM), flash memory, or any other memory technology. In some examples, the memory 414 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 412 and/or the external defibrillator 400. In some cases, the memory 414 at least temporarily stores the ECG signal and/or the impedance signal.

In various examples, the memory 414 includes a detector 416, which causes the processor(s) 412 to determine, based on the ECG signal and/or the impedance signal, whether the individual 408 is exhibiting a particular heart rhythm. For instance, the processor(s) 412 determines whether the individual 408 is experiencing a shockable rhythm that is treatable by defibrillation. Examples of shockable rhythms include ventricular fibrillation (VF) and ventricular tachycardia (VT). In some examples, the processor(s) 412 determines whether any of a variety of different rhythms (e.g., asystole, sinus rhythm, atrial fibrillation (AF), etc.) are present in the ECG signal.

The processor(s) 412 is operably connected to one or more input devices 418 and one or more output devices 420. Collectively, the input device(s) 418 and the output device(s) 420 function as an interface between a user and the defibrillator 400. The input device(s) 418 is configured to receive an input from a user and includes at least one of a keypad, a cursor control, a touch-sensitive display, a voice input device (e.g., a microphone), a haptic feedback device (e.g., a gyroscope), one or more physiological parameters, or any combination thereof. The output device(s) 420 includes at least one of a display, a speaker, a haptic output device, a printer, or any combination thereof. In particular cases, the output device(s) 420 include a light bar 421 configured to output coordinated visual feedback to one or more users. In various examples, the processor(s) 412 causes a display among the input device(s) 418 to visually output a waveform of the ECG signal and/or the impedance signal. In some implementations, the input device(s) 418 includes one or more touch sensors, the output device(s) 420 includes a display screen, and the touch sensor(s) are integrated with the display screen. Thus, in some cases, the external defibrillator 400 includes a touchscreen configured to receive user input signal(s) and visually output physiological parameters, such as the ECG signal and/or the impedance signal.

In various implementations, the input device(s) 418 further include, or are otherwise connected to, one or more physiological sensors. The physiological sensor(s), for instance, are configured to detect one or more physiological parameters of the individual 408. Examples of the physiological sensor(s) include a blood pressure sensor (e.g., a blood pressure cuff, invasive blood pressure sensor, or the like), an airway sensor (e.g., a sensor configured to detect a partial pressure of CO₂ and/or O₂ in an airway of the individual 408), a blood oxygenation sensor (e.g., a pulse oximeter, regional oxygenation sensor, or the like), a thermometer, a pulse sensor, a blood flow sensor (e.g., an ultrasound transducer configured to detect blood flow using Doppler-based techniques), an airway pressure sensor, or any combination thereof. The input device(s) 418, in some cases, includes one or more sensors configured to detect other characteristics of the individual 408. For example, the input device(s) 418 includes an accelerometer, gyroscope, microphone, or any combination thereof. In various implementations, the processor(s) 412 is configured to assess a condition of the individual 408 by analyzing data derived from signals detected by the input device(s) 418.

In some examples, the memory 414 includes an advisor 422, which, when executed by the processor(s) 412, causes the processor(s) 412 to generate advice and/or control the output device(s) 420 to output the advice to a user (e.g., a rescuer). In some examples, the processor(s) 412 provides, or causes the output device(s) 420 to provide, an instruction to perform CPR on the individual 408. In some cases, the processor(s) 412 evaluates, based on the ECG signal, the impedance signal, or other physiological parameters, CPR being performed on the individual 408 and causes the output device(s) 420 to provide feedback about the CPR in the instruction. According to some examples, the processor(s) 412, upon identifying that a shockable rhythm is present in the ECG signal, causes the output device(s) 420 to output an instruction and/or recommendation to administer a defibrillation shock to the individual 408.

The memory 414 also includes an initiator 424 which, when executed by the processor(s) 412, causes the processor(s) 412 to control other elements of the external defibrillator 400 in order to administer a defibrillation shock to the individual 408. In some examples, the processor(s) 412 executing the initiator 424 selectively causes the administration of the defibrillation shock based on determining that the individual 408 is exhibiting the shockable rhythm and/or based on an input from a user (received, e.g., by the input device(s) 418. In some cases, the processor(s) 412 causes the defibrillation shock to be output at a particular time, which is determined by the processor(s) 412 based on the ECG signal and/or the impedance signal.

The processor(s) 412 is operably connected to a charging circuit 423 and a discharge circuit 425. In various implementations, the charging circuit 423 includes a power source 426, one or more charging switches 428, and one or more capacitors 430. The power source 426 includes, for instance, a battery. The processor(s) 412 initiates a defibrillation shock by causing the power source 426 to charge at least one capacitor among the capacitor(s) 430. For example, the processor(s) 412 activates at least one of the charging switch(es) 428 in the charging circuit 423 to complete a first circuit connecting the power source 426 and the capacitor to be charged. Then, the processor(s) 412 causes the discharge circuit 425 to discharge energy stored in the charged capacitor across a pair of defibrillation electrodes 434, which are in contact with the individual 408. For example, the processor(s) 412 deactivates the charging switch(es) 428 completing the first circuit between the capacitor(s) 430 and the power source 426, and activates one or more discharge switches 432 completing a second circuit connecting the charged capacitor 430 and at least a portion of the individual 408 disposed between defibrillation electrodes 434.

The energy is discharged from the defibrillation electrodes 434 in the form of a defibrillation shock. For example, the defibrillation electrodes 434 are connected to the skin of the individual 408 and located at positions on different sides of the heart of the individual 408, such that the defibrillation shock is applied across the heart of the individual 408. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 432 are controlled by the processor(s) 412, for example. In various implementations, the defibrillation electrodes 434 are connected to defibrillation wires 436. The defibrillation wires 436 are connected to a defibrillation port 438, in implementations. According to various examples, the defibrillation wires 436 are removable from the defibrillation port 438. For example, the defibrillation wires 436 are plugged into the defibrillation port 438.

In various implementations, the processor(s) 412 is operably connected to one or more transceivers 440 that transmit and/or receive data over one or more communication networks 442. For example, the transceiver(s) 440 includes a network interface card (NIC), a network adapter, a local area network (LAN) adapter, or a physical, virtual, or logical address to connect to the various external devices and/or systems. In various examples, the transceiver(s) 440 includes any sort of wireless transceivers capable of engaging in wireless communication (e.g., radio frequency (RF) communication). For example, the communication network(s) 442 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 440 includes other wireless modems, such as a modem for engaging in WI-FI^{®}, WIGIG^{®}, WIMAX^{®}, BLUETOOTH^{®}, or infrared communication over the communication network(s) 442.

The defibrillator 400 is configured to transmit and/or receive data (e.g., ECG data, impedance data, data indicative of one or more detected heart rhythms of the individual 408, data indicative of one or more defibrillation shocks administered to the individual 408, etc.) with one or more external devices 444 via the communication network(s) 442. The external devices 444 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 442. In some examples, the external device(s) 444 is located remotely from the defibrillator 400, such as at a remote clinical environment (e.g., a hospital). According to various implementations, the processor(s) 412 causes the transceiver(s) 440 to transmit data to the external device(s) 444. In some cases, the transceiver(s) 440 receives data from the external device(s) 444 and the transceiver(s) 440 provide the received data to the processor(s) 412 for further analysis.

In various implementations, the external defibrillator 400 also includes a housing 446 that at least partially encloses other elements of the external defibrillator 400. For example, the housing 446 encloses the detection circuit 410, the processor(s) 412, the memory 414, the charging circuit 423, the transceiver(s) 440, or any combination thereof. In some cases, the input device(s) 418 and output device(s) 420 extend from an interior space at least partially surrounded by the housing 446 through a wall of the housing 446. In various examples, the housing 446 acts as a barrier to moisture, electrical interference, and/or dust, thereby protecting various components in the external defibrillator 400 from damage.

According to some examples, the memory 414 stores instructions for executing a generator 448. The generator 448, when executed by the processor(s) 412, causes the external defibrillator 400 to generate directions, timing information, output signals, and communication signals, as described herein. For instance, the generator 448 enables the external defibrillator 400 to coordinate feedback output by the external defibrillator 400 (e.g., using the light bar 421) and one or more of the external device(s) 444.

In some implementations, the external defibrillator 400 is an automated external defibrillator (AED) operated by an untrained user (e.g., a bystander, layperson, etc.) and can be operated in an automatic mode. In automatic mode, the processor(s) 412 automatically identifies a rhythm in the ECG signal, makes a decision whether to administer a defibrillation shock, charges the capacitor(s) 430, discharges the capacitor(s) 430, or any combination thereof. In some cases, the processor(s) 412 controls the output device(s) 420 to output (e.g., display) a simplified user interface to the untrained user. For example, the processor(s) 412 refrains from causing the output device(s) 420 to display a waveform of the ECG signal and/or the impedance signal to the untrained user, in order to simplify operation of the external defibrillator 400.

In some examples, the external defibrillator 400 is a monitor-defibrillator utilized by a trained user (e.g., a clinician, an emergency responder, etc.) and can be operated in a manual mode or the automatic mode. When the external defibrillator 400 operates in manual mode, the processor(s) 412 cause the output device(s) 420 to display a variety of information that may be relevant to the trained user, such as waveforms indicating the ECG data and/or impedance data, notifications about detected heart rhythms, and the like.

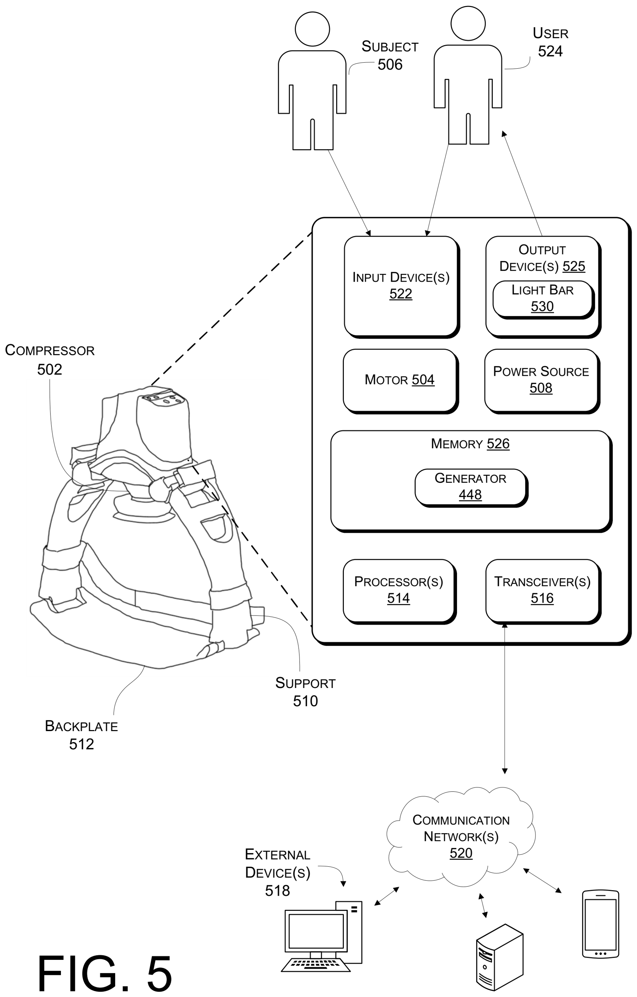

FIG. 5 illustrates a chest compression device 500 configured to perform various functions described herein. For example, the chest compression device 500 is the chest compression device 106 described in reference to FIG. 1.

In various implementations, the chest compression device 500 includes a compressor 502 that is operatively coupled to a motor 504. The compressor 502 physically administers a force to the chest of a subject 506 that compresses the chest of the subject 506. In some examples, the compressor 502 includes at least one piston that periodically moves between two positions (e.g., a compressed position and a release position) at a compression frequency. For example, when the piston is positioned on the chest of the subject 506, the piston compresses the chest when the piston is moved into the compressed position. A suction cup may be positioned on a tip of the piston, such that the suction cup contacts the chest of the subject 506 during operation. In various cases, the compressor 502 includes a band that periodically tightens to a first tension and loosens to a second tension at a compression frequency. For instance, when the band is disposed around the chest of the subject 506, the band compresses the chest when the band tightens.

The motor 504 is configured to convert electrical energy stored in a power source 508 into mechanical energy that moves and/or tightens the compressor 502, thereby causing the compressor 502 to administer the force to the chest of the subject 506. In various implementations, the power source 508 is portable. For instance, the power source 508 includes at least one rechargeable (e.g., lithium-ion) battery. In some cases, the power source 508 supplies electrical energy to one or more elements of the chest compression device 500 described herein.

In various cases, the chest compression device 500 includes a support 510 that is physically coupled to the compressor 502, such that the compressor 502 maintains a position relative to the subject 506 during operation. In some implementations, the support 510 is physically coupled to a backplate 512, cot, or other external structure with a fixed position relative to the subject 506. According to some cases, the support 510 is physically coupled to a portion of the subject 506, such as wrists of the subject 506.

The operation of the chest compression device 500 may be controlled by at least one processor 514. In various implementations, the motor 504 is communicatively coupled to the processor(s) 514. Specifically, the processor(s) 514 is configured to output a control signal to the motor 504 that causes the motor 504 to actuate the compressor 502. For instance, the motor 504 causes the compressor 502 to administer the compressions to the subject 506 based on the control signal. In some cases, the control signal indicates one or more treatment parameters of the compressions. Examples of treatment parameters include a frequency, timing, depth, force, position, velocity, and acceleration of the compressor 502 administering the compressions. According to various cases, the control signal causes the motor 504 to cease compressions.

In various implementations, the chest compression device 500 includes at least one transceiver 516 configured to communicate with at least one external device 518 over one or more communication networks 520. Any communication network described herein can be included in the communication network(s) 520 illustrated in FIG. 5. The external device(s) 518, for example, includes at least one of a monitor-defibrillator, an AED, an ECMO device, a ventilation device, a patient monitor, a mobile phone, a server, or a computing device. In some implementations, the transceiver(s) 516 is configured to communicate with the external device(s) 518 by transmitting and/or receiving signals wirelessly. For example, the transceiver(s) 516 includes a NIC, a network adapter, a LAN adapter, or a physical, virtual, or logical address to connect to the various external devices and/or systems. In various examples, the transceiver(s) 516 includes any sort of wireless transceivers capable of engaging in wireless communication (e.g., RF communication). For example, the communication network(s) 520 includes one or more wireless networks that include a 3GPP network, such as an LTE RAN (e.g., over one or more LTE bands), an NR RAN (e.g., over one or more NR bands), or a combination thereof. In some cases, the transceiver(s) 516 includes other wireless modems, such as a modem for engaging in WI-FI^{®}, WIGIG^{®}, WIMAX^{®}, BLUETOOTH^{®}, or infrared communication over the communication network(s) 520. The signals, in various cases, encode data in the form of data packets, datagrams, or the like. In some cases, the signals are transmitted as compressions are being administered by the chest compression device 500 (e.g., for real-time feedback by the external device(s) 518), after compressions are administered by the chest compression device 500 (e.g., for post-event review at the external device 518), or a combination thereof.

In various cases, the processor(s) 514 generates the control signal based on data encoded in the signals received from the external device(s) 518. For instance, the signals include an instruction to initiate the compressions, and the processor(s) 514 instructs the motor 504 to begin actuating the compressor 502 in accordance with the signals.

In some cases, the chest compression device 500 includes at least one input device 522. In various examples, the input device(s) 522 is configured to receive an input signal from a user 524, who may be a rescuer treating the subject 506. Examples of the input device(s) 522 include, for instance, at a keypad, a cursor control, a touch-sensitive display, a voice input device (e.g., a microphone), a haptic feedback device (e.g., a gyroscope), one or more physiological sensors, or any combination thereof. In various implementations, the processor(s) 514 generate the control signal based on the input signal. For instance, the processor(s) 514 generate the control signal to adjust a frequency of the compressions based on the chest compression device 500 detecting a selection by the user 524 of a user interface element displayed on a touchscreen or detecting the user 524 pressing a button integrated with an external housing of the chest compression device 500.

According to some examples, the input device(s) 522 include one or more sensors. The sensor(s), for example, is configured to detect a physiological parameter of the subject 506. In some implementations, the sensor(s) is configured to detect a state parameter of the chest compression device 500, such as a position of the compressor 502 with respect to the subject 506 or the backplate 512, a force administered by the compressor 502 on the subject 506, a force administered onto the backplate 512 by the body of the subject 506 during a compression, or the like. According to some implementations, the signals transmitted by the transceiver(s) 516 indicate the physiological parameter(s) and/or the state parameter(s).

The chest compression device 500 further includes at least one output device 525, in various implementations. Examples of the output device(s) 525 include, for instance, least one of a display (e.g., a projector, an LED screen, etc.), a speaker, a haptic output device, a printer, or any combination thereof. In some implementations, the output device(s) 525 include a screen configured to display various parameters detected by and/or reported to the chest compression device 500, a charge level of the power source 508, a timer indicating a time since compressions were initiated or paused, and other relevant information. According to some examples, the output device(s) 525 include a light bar 530 configured to output coordinated feedback to the user 524 and/or one or more additional users.

The chest compression device 500 further includes memory 526. In various implementations, the memory 526 is volatile (such as random access memory (RAM)), non-volatile (such as read only memory (ROM), flash memory, etc.) or some combination of the two. The memory 526 stores instructions that, when executed by the processor(s) 514, causes the processor(s) 514 to perform various operations. In various examples, the memory 526 stores methods, threads, processes, applications, objects, modules, any other sort of executable instruction, or a combination thereof. In some cases, the memory 526 stores files, databases, or a combination thereof. In some examples, the memory 526 includes, but is not limited to, RAM, ROM, EEPROM, flash memory, or any other memory technology. In some examples, the memory 526 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 526 stores instructions, programs, threads, objects, data, or any combination thereof, that cause the processor(s) 514 to perform various functions. In various cases, the memory 526 stores one or more parameters that are detected by the chest compression device 500 and/or reported to the chest compression device 500. In implementations of the present disclosure, the memory 526 also stores instructions for executing the generator 448.

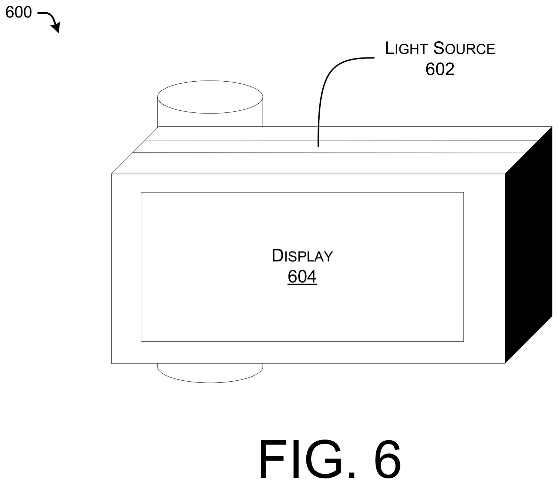

FIG. 6 illustrates an example of a monitor 600 of a ventilation device configured to provide coordinated feedback. For example, the monitor 600 includes a light source 602 disposed adjacent to a display 604 of the monitor 600. In this example, the light source 602 is disposed on sides of the monitor 600 that extend from a side on which the display 604 is disposed. In various cases, the light source 602 is wrapped around the monitor 600 and is configured to output light in multiple directions, at least one of which is different than a direction in which the display 604 outputs visual signals. Upon generating and/or receiving instructions, the monitor 600 may be configured to provide coordinated feedback by illuminating the light source 602 in a manner consistent with one or more characteristics (e.g., a color, timing, frequency, duty cycle, indicated by the instructions.

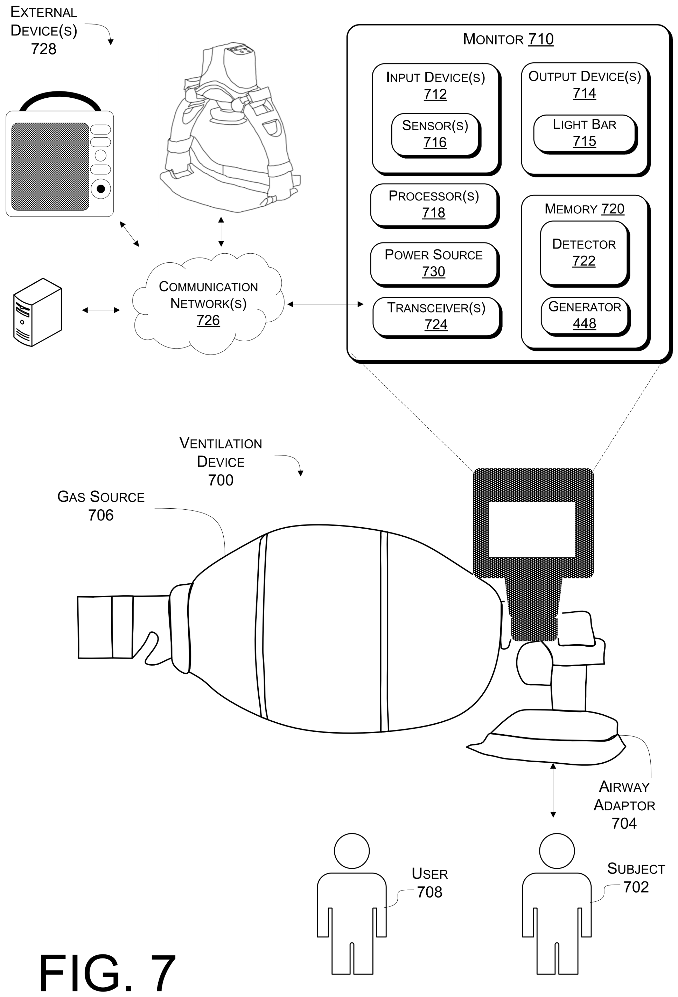

FIG. 7 illustrates a ventilation device 700 configured to perform various functions described herein. For instance, the ventilation device 700 is the ventilation device 108 described above with reference to FIG. 1.

The ventilation device 700 is configured to provide assisted ventilation to a subject 702. The ventilation device 700 includes an airway adaptor 704 configured to be fluidically coupled with an airway of the subject 702. In some implementations, the airway adaptor 704 includes a mask configured to be disposed on a face of the subject 702. For example, the airway adaptor 704 may be pressed over the mouth onto the face of the subject 702. In some implementations, the airway adaptor 704 is inserted into the mouth of the subject 702. For example, the airway adaptor 704, in some cases, includes a supraglottic airway adaptor (also referred to as a "supraglottic airway") configured to be disposed in a pharynx of the subject 702. In some cases, the airway adaptor 704 includes an extraglottic device, such as a laryngeal tube, a pharyngeal tube, a Combitube (also referred to as an "esophageal tracheal airway"), or any combination thereof. In some cases, the airway adaptor 704 includes an endotracheal tube. In various implementations, the airway adaptor 704 is configured to form a fluid-tight seal with a fluid circuit that includes the airway of the subject 702. In some examples, the airway adaptor 704 is disposable.

In various implementations, the ventilation device 700 further includes a gas source 706 configured to control a flow of a gas into the airway adaptor 704 and/or a flow of gas out of the airway adaptor 704. The gas source 706 is configured to be fluidically coupled with the airway adaptor 704. For instance, the gas source 706 is removably coupled with the airway adaptor 704. In cases in which the airway adaptor 704 is fluidically coupled with the airway of the subject 702, the gas source 706 is configured to control the flow of gas (e.g., air, oxygen, carbon dioxide, gaseous medications, or any combination thereof) into the airway and/or out of the airway. In some cases, the ventilation device 700 performs positive pressure ventilation (PPV) on the subject 702. For example, the gas source 706 is configured to induce a higher pressure within the gas source 706 than the airway of the subject 702, thereby pushing a gas into the airway of the subject 702 through the airway adaptor 704. In some cases, the gas source 706 is configured to induce a lower pressure within the gas source 706 than the airway of the subject 702, thereby pulling a gas from the airway of the subject 702 into the gas source 706 through the airway adaptor 704. The gas source 706 may be at least partially reusable, at least partially disposable, or a combination thereof.

In some implementations, the gas source 706 operates automatically. For instance, the gas source 706 may include a mechanical ventilator configured to control the gas delivered to and from the airway of the subject 702 through the airway adaptor 704.

In some cases, the gas source 706 is manually operated. For instance, the gas source 706 may include a bag that is manually squeezed and released by a user 708. When the bag is squeezed (e.g., by the hands of the user 708), an interior pressure within the gas source 706 increases (e.g., producing a net flow of gas into the airway of the subject 702). When the bag is released, the interior pressure within the gas source 706 decreases (e.g., producing a net flow of gas out of the airway of the subject 702). Accordingly, the user 708 may be configured to control a rate of the assisted ventilation corresponding to the rate at which the user 708 squeezes and releases the bag. Moreover, the user 708 may be configured to control a volume of gas delivered into the airway and lungs of the subject 702 based on an amount of volume of gas that the user 708 squeezes out of the bag. In various cases, the user 708 may be configured to control a pressure of gas in the fluid circuit between the airway of the subject 702, the airway adaptor 704, and the gas source 706 based on a force at which the user 708 is squeezing the bag. The user 708 may additionally control a flow rate of gas through the fluid circuit by controlling a rapidity by which the user 708 squeezes the bag.

In some cases, the gas source 706 is selectively vented to an external environment containing air. In some examples, the gas source 706 includes a cannister, bag, or other receptacle containing a gas (e.g., oxygen) to be delivered to the airway of the subject 702. In some cases, the gas source 706 can be coupled with a source of a medication (e.g., albuterol) that can be delivered to the subject 702 by the delivery of the assisted ventilation. One or more valves can be disposed in the gas source 706 to control a type and/or amount of gas delivered to the subject 702.

In some examples, the ventilation device 700 includes one or more valves configured to control a direction of gas flow between the airway adaptor 704 and the gas source 706. For example, a valve may be coupled between the airway adaptor 704 and the gas source 706. In some cases, the valve is configured to open during an inspiratory phase (e.g., the pressure in the gas source 706 is greater than the pressure in the airway adaptor 704), thereby allowing the gas to flow from the gas source 706 to the airway adaptor 704. According to some cases, a valve (e.g., the same or a different valve) is coupled between the airway adaptor 704 and an expiratory port (e.g., exposed to an external environment). During an expiratory phase (e.g., the pressure in the airway adaptor 704 is greater than the pressure in the gas source 706), the valve may vent the fluid circuit to the expiratory port. Suitable valves include shutter valves, duckbill valves, and the like.

In some implementations, the ventilation device 700 further includes a monitor 710 configured to monitor the subject 702 and to otherwise assist the user 708 with operating the ventilation device 700. In some cases, the monitor 710 is a reusable device that is configured to be clipped, inserted over, or otherwise disposed on a portion of the fluid circuit including the airway adaptor 704 and the gas source 706. For example, the monitor 710 is configured to be removably coupled with an intermediary tube connecting the airway adaptor 704 to the gas source 706.

The monitor 710 includes one or more input devices 712 and one or more output devices 714. Collectively, the input device(s) 712 and the output device(s) 714 function as an interface between the monitor 710 and the subject 702 and/or between the monitor 710 and the user 708. In some cases, the input device(s) 712 are configured to receive input signals from the user 708. For instance, the input device(s) 712 include at least one of a keypad, a cursor control, a touch-sensitive display, a voice input device (e.g., a microphone), a haptic feedback device (e.g., a gyroscope), or any combination thereof. The output device(s) 714 include at least one of a display (e.g., a screen, one or more light sources, etc.), a speaker, a haptic output device, a printer, or any combination thereof. In some implementations, the input device(s) 712 include one or more touch sensors, the output device(s) 714 include a display screen, and the touch sensor(s) are integrated with the display screen. Thus, in some cases, the monitor 710 includes a touchscreen configured to receive user input signal(s) and visually output information. The output device(s) 714, in various cases, further include a light bar 715 configured to output coordinated visual feedback to one or more users including the user 708.

In various implementations, the input device(s) 712 further include, or are otherwise connected to, one or more sensors 716. The sensor(s) 716, for instance, are configured to detect one or more parameters. In some cases, the parameters are characteristics of the fluid circuit including the airway adaptor 704 and the gas source 706. In some implementations, the parameters include physiological parameters of the subject 702. According to some cases, the parameters include operation characteristics of the ventilation device 700 by the user 708. In various implementations, the sensor(s) 716 include at least one pressure sensor (e.g., configured to detect a pressure in the fluid circuit), at least one gas sensor (e.g., a nondispersive infrared sensor configured to detect a partial pressure of CO₂ in the fluid circuit, an oxygen sensor configured to detect an amount of oxygen in the fluid circuit, or the like), at least one flow sensor (e.g., an ultrasound sensor or an interferometric sensor configured to detect a velocity or flow rate of gas in the fluid circuit), at least one humidity sensor (e.g., configured to detect a humidity in the fluid circuit), at least one temperature sensor (e.g., configured to detect a temperature in the fluid circuit), at least one accelerometer, at least one gyroscope, at least one microphone (e.g., configured to detect breath sounds of the subject 702), or any combination thereof.

The monitor 710 further includes at least one processor 718. In some implementations, the processor(s) 718 includes a central processing unit (CPU), a graphics processing unit (GPU), both CPU and GPU, or other processing unit or component known in the art.

The processor(s) 718 is operably connected to memory 720. In various implementations, the memory 720 is volatile (such as random access memory (RAM)), non-volatile (such as read only memory (ROM), flash memory, etc.) or some combination of the two. The memory 720 stores instructions that, when executed by the processor(s) 718, causes the processor(s) 718 to perform various operations. In various examples, the memory 720 stores methods, threads, processes, applications, objects, modules, any other sort of executable instruction, or a combination thereof. In some cases, the memory 720 stores files, databases, or a combination thereof. In some examples, the memory 720 includes, but is not limited to, RAM, ROM, electrically erasable programmable read-only memory (EEPROM), flash memory, or any other memory technology. In some examples, the memory 720 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 718 and/or the monitor 710.

In various implementations, the processor(s) 718 are configured to assess a condition of the subject 702 by analyzing data derived from signals detected by the input device(s) 712. For example, the memory 720 stores a detector 722 that, when executed by the processor(s) 718, causes the processor(s) 718 to determine one or more physiological parameters of the subject 702 based on the signals detected by the sensor(s) 716. For instance, the processor(s) 718 may determine an airway pressure of the subject 702 by analyzing the pressure in the fluid circuit, a partial pressure of CO₂ (e.g., capnograph) of the subject 702 by analyzing a partial pressure of CO₂ in the fluid circuit, a lung volume of the subject 702 by analyzing the flow rate of gas in the fluid circuit, a respiratory rate of the subject 702 by analyzing one or more parameters of the fluid circuit, or any combination thereof. In some cases, the processor(s) 718 may determine whether the subject 702 is spontaneously breathing by one or more parameters of the fluid circuit. The processor(s) 718, in some cases, generates one or more alerts in response to detecting that one or more of the physiological parameters are outside of one or more threshold ranges.

According to some cases, the processor(s) 718 are further configured to identify a condition associated with operation of the ventilation device 700 by the user 708 based on one or more signals detected by the input device(s) 712. For example, upon executing the detector 722, the processor(s) 718 may be configured to detect a ventilation rate, a ventilation volume, a ventilation pressure, or the like, applied to the subject 702. In some cases, the processor(s) 718 are configured to detect one or more potential problems in operation of the ventilation device 700. For example, the processor(s) 718 may detect that the ventilation rate, volume, or pressure is outside of a threshold range, that there is a leak between the airway of the subject 702 and the airway adaptor 704, that the subject 702 is being hyperventilated, that the subject 702 is being hypoventilated, or any combination thereof.

In various implementations, the processor(s) 718 is configured to generate feedback based on the signals detected by the input devices 712, the condition of the subject 702, or the condition associated with operation of the ventilation device 700. In some cases, the processor(s) 718 cause the output device(s) 714 to output indications of the feedback to the user 708. Thus, the user 708 may be appraised of the condition of the subject 702 and/or of the quality of ventilation being applied to the subject 702. In some cases, the feedback is provided to the user 708 substantially in real-time. In implementations of the present disclosure, the memory 720 also stores instructions for executing the generator 448.

The monitor 710 further includes one or more transceivers 724 that transmit and/or receive data over one or more communication networks 726. For example, the transceiver(s) 724 includes a network interface card (NIC), a network adapter, a local area network (LAN) adapter, or a physical, virtual, or logical address to connect to the various external devices and/or systems. In various examples, the transceiver(s) 724 includes any sort of wireless transceivers capable of engaging in wireless communication (e.g., radio frequency (RF) communication). For example, the communication network(s) 726 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 724 includes other wireless modems, such as a modem for engaging in WI-FI^{®}, WIGIG^{®}, WIMAX^{®}, BLUETOOTH^{®}, or infrared communication over the communication network(s) 726.

The monitor 710 is configured to transmit and/or receive data (e.g., one or more parameters of the fluid circuit, one or more physiological parameters of the subject 702, one or more parameters associated with operation of the ventilation device 700, feedback related to the condition of the subject 702, feedback related to operation of the ventilation device 700, etc.) with one or more external devices 728 via the communication network(s) 726. The external devices 728 include, for instance, mobile devices (e.g., mobile phones, smart watches, etc.), Internet of Things (IoT) devices, medical devices (e.g., monitor-defibrillators, AEDs, mechanical chest compression devices, etc.), computers (e.g., laptop devices, servers, etc.), or any other type of computing device configured to communicate over the communication network(s) 726. In some examples, the external device(s) 728 are located remotely from the ventilation device 700, such as at a remote clinical environment (e.g., a hospital). According to various implementations, the processor(s) 718 cause the transceiver(s) 724 to transmit data to the external device(s) 728. In some cases, the transceiver(s) 724 receives data from the external device(s) 728 and the transceiver(s) 724 provide the received data to the processor(s) 718 for further analysis.

In various implementations, the monitor 710 further includes a power source 730. The power source 730 is configured to store power that can be distributed to various components of the monitor 710. For instance, the power source 730 is configured to supply power to the input device(s) 712, the output device(s) 714, the sensor(s) 716, the processor(s) 718, the memory 720, the transceiver(s) 724, or any combination thereof. In some cases, the monitor 710, including the power source 730, is a portable electronic device. Examples of the power source 730 include at least one battery, at least one capacitor, at least one power generator (e.g., an antenna configured to induce a current in a circuit in response to receiving an electromagnetic signal from an external device), or any combination thereof. In some cases, the power source 730 is disposable. In some examples, the power source 730 is reusable and rechargeable.

### EXAMPLE CLAUSES

The following clauses provide various examples of the present disclosure:
1. A medical device system, including: a monitor-defibrillator configured to receive a ventilation instruction, the monitor-defibrillator including a first light source configured to output a first light signal indicating the ventilation instruction; a chest compression device configured to receive the ventilation instruction, the chest compression device including a second light source configured to output a second light signal indicating the ventilation instruction, the second light signal being output by the second light source simultaneously when the first light source outputs the first light signal; and a ventilation device including: a sensor configured to detect an airway parameter of a subject; a processor configured to generate the ventilation instruction by analyzing the airway parameter; a transceiver configured to output the ventilation instruction; and a third light source configured to output a third light signal indicating the ventilation instruction, the third light signal being output by the third light source simultaneously when the first light source outputs the first light signal and the second light source outputs the second light signal.
2. The medical device system of clause 1, wherein the monitor-defibrillator includes a first display that is separate from the first light source, and wherein the ventilation device includes a second display that is separate from the second light source.
3. The medical device system of clause 1 or 2, wherein the first light signal, the second light signal, and the third light signal have a common color.
4. A system, including: a first medical device including: a processor configured to generate an instruction or an alert by analyzing a physiological parameter of a subject; a first light source configured to output a first light signal indicating the instruction or the alert; and a first transceiver configured to output a communication signal indicating the instruction or the alert; and a second medical device including: a second transceiver configured to receive the communication signal indicating the instruction or the alert; a second light source configured to output a second light signal indicating the instruction or the alert, the first light signal and the second light signal having a common characteristic.
5. The system of clause 4, wherein the first medical device or the second medical device includes a ventilation device, and wherein the first medical device or the second medical device includes: a monitor-defibrillator; an automated external defibrillator (AED); or a chest compression device.
6. The system of clause 4 or 5, wherein: the instruction is to administer a treatment to the subject, the treatment including assisted ventilation, administration of an electrotherapy, administration of a medication, or administration of chest compressions.
7. The system of clause 6, wherein: the instruction is to administer the treatment at a particular time; the first light source is configured to output the first light signal at the particular time; and the second light source is configured to output the second light signal at the particular time.
8. The system of clause 6 or 7, the treatment being a first treatment, wherein the instruction is further to refrain from administering a second treatment to the subject.
9. The system of any of clauses 4 to 8, wherein: the processor is configured to generate the instruction or the alert by: determining, by analyzing the physiological parameter, that the subject has a condition including spontaneous circulation, lack of spontaneous circulation, spontaneous respiration, lack of spontaneous respiration, ineffectiveness of a paralytic, a cardiac arrhythmia, or resolution of a cardiac arrhythmia; and generating the instruction by determining a treatment or an alteration of a treatment that addresses the condition.
10. The system of any of clauses 4 to 9, wherein: the processor is configured to generate the instruction or the alert by comparing the physiological parameter to a threshold.
11. The system of any of clauses 4 to 10, the communication signal being a first communication signal, wherein: the second transceiver is configured to transmit a second communication signal; the first transceiver is configured to receive the second communication signal; and the first light source is further configured to output a third light signal in response to the first transceiver receiving the second communication signal, the third light signal indicating that the first medical device is communicatively coupled with the second medical device.
12. The system of any of clauses 4 to 11, wherein the common characteristic includes a common timing, a common color, a common pulse pattern, or a common brightness.
13. The system of any of clauses 4 to 12, the instruction being a first instruction, the alert being a first alert, the common characteristic being a first color, wherein: the second transceiver is configured to transmit a second communication signal indicating a second instruction or a second alert; the first transceiver is configured to receive the second communication signal; the first light source is configured to output a third light signal indicating the second instruction or the second alert; the second light source is configured to output a fourth light signal indicating the second instruction or the second alert, the fourth light signal being output by the second light source at a same time as the third light signal; and the third light signal and the fourth light signal have a second color that is different than the first color.
14. The system of clause 13, wherein: the first medical device further includes a battery, and wherein the second alert indicates that a charge level of the battery is lower than a threshold.
15. The system of clause 13 or 14, wherein the second alert indicates whether the first medical device is communicatively coupled with a third medical device.
16. The system of any of clauses 4 to 15, wherein the first medical device further includes a sensor configured to detect the physiological parameter of the subject.
17. The system of clause 16, the communication signal being a first communication signal, wherein: the sensor is configured to generate a first estimate of the physiological parameter of the subject; the first transceiver is configured to receive, from an external device, a second communication signal indicating a second estimate of the physiological parameter of the subject; and the processor is configured to: select, among the first estimate and the second estimate, a prioritized estimate of the physiological parameter; and generate the instruction or the alert by analyzing the prioritized estimate of the physiological parameter.
18. The system of any of clauses 4 to 17, wherein the processor is configured to: analyze the physiological parameter by determining that a condition of the subject has deteriorated; and generate the instruction or alert in response to determining that the condition of the subject has deteriorated.
19. A method, including: generating, by a first device, an instruction or an alert; outputting, by the first device, a first output signal indicating the instruction or the alert; transmitting, by the first device to a second device, a communication signal indicating the instruction or the alert; outputting, by the second device, a second output signal indicating the instruction or the alert, the second output signal having a common characteristic with the first output signal.
20. The method of clause 19, wherein the first device includes a first medical device or the second device includes a second medical device.
21. The method of clause 19 or 20, wherein generating, by the first device, the instruction or the alert includes: determining that a physiological parameter is outside of a first range; determining that a treatment parameter is outside of a second range; determining that a charge level of a battery of the first device is below a threshold; or determining whether the first device is communicatively coupled with a third device.
22. The method of clause 21, wherein generating, by the first device, the instruction or the alert includes: determining, that a subject has a condition including spontaneous circulation, lack of spontaneous circulation, spontaneous respiration, lack of spontaneous respiration, ineffectiveness of a paralytic, a cardiac arrhythmia, or resolution of a cardiac arrhythmia, and generating the instruction to indicate a treatment or an alteration of a treatment that addresses the condition.
23. The method of any of clauses 19 to 22, wherein the instruction is to administer a treatment to a subject, the treatment including assisted ventilation, administration of an electrotherapy, administration of a medication, or administration of chest compressions.
24. The method of clause 23, wherein: the instruction is to administer the treatment at a particular time; the first device outputs the first output signal at the particular time; and the second device outputs the second output signal at the particular time.
25. The method of clause 23 or 24, the treatment being a first treatment, wherein the instruction is further to refrain from administering a second treatment to the subject.
26. The method of any of clauses 19 to 25, wherein the common characteristic includes a common timing, a common wavelength, a common pulse pattern, or a common intensity.
27. The method of any of clauses 19 to 26, the instruction being a first instruction, the alert being a first alert, the common characteristic being a first wavelength, wherein the method further includes: transmitting, by the second device to the first device, a second communication signal indicating a second instruction or a second alert; outputting, by the first device, a third output signal indicating the second instruction or the second alert; and outputting, by the second device, a fourth output signal indicating the second instruction or the second alert, the second device outputting the fourth output signal at a same time as the first device outputting the third output signal, the third output signal and the fourth output signal having a second wavelength that is different than the first wavelength.
28. The method of any of clauses 19 to 27, wherein: the first output signal includes a first light signal, the second output signal includes a second light signal, and the first light signal has a same wavelength as the second light signal.
29. The method of any of clauses 19 to 28, further including: determining that the first device and the second device are associated with a same subject.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11 % of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A system, comprising:
a first medical device comprising:
a processor configured to generate an instruction or an alert by analyzing a physiological parameter of a subject;
a first light source configured to output a first light signal indicating the instruction or the alert; and
a first transceiver configured to output a communication signal indicating the instruction or the alert; and
a second medical device comprising:
a second transceiver configured to receive the communication signal indicating the instruction or the alert;
a second light source configured to output a second light signal indicating the instruction or the alert, the first light signal and the second light signal having a common characteristic.

2. The system of claim 1, wherein the first medical device or the second medical device comprises a ventilation device, and
wherein the first medical device or the second medical device comprises:
a monitor-defibrillator;
an automated external defibrillator (AED); or
a chest compression device.

3. The system of claim 1 or 2, wherein:
the instruction is to administer a treatment to the subject, the treatment comprising assisted ventilation, administration of an electrotherapy, administration of a medication, or administration of chest compressions.

4. The system of claim 3, wherein:
the instruction is to administer the treatment at a particular time;
the first light source is configured to output the first light signal at the particular time; and
the second light source is configured to output the second light signal at the particular time.

5. The system of claim 3 or 4, the treatment being a first treatment, wherein the instruction is further to refrain from administering a second treatment to the subject.

6. The system of any one of claims 1-5, wherein:
the processor is configured to generate the instruction or the alert by:
determining, by analyzing the physiological parameter, that the subject has a condition comprising spontaneous circulation, lack of spontaneous circulation, spontaneous respiration, lack of spontaneous respiration, ineffectiveness of a paralytic, a cardiac arrhythmia, or resolution of a cardiac arrhythmia; and
generating the instruction by determining a treatment or an alteration of a treatment that addresses the condition.

7. The system of any one of claim 1-6, wherein the common characteristic comprises a common timing, a common color, a common pulse pattern, or a common brightness.

8. The system of any one of claims 1-7, wherein the first medical device further comprises a sensor configured to detect the physiological parameter of the subject.

9. A method, comprising:
generating, by a first device, an instruction or an alert;
outputting, by the first device, a first output signal indicating the instruction or the alert;
transmitting, by the first device to a second device, a communication signal indicating the instruction or the alert;
outputting, by the second device, a second output signal indicating the instruction or the alert, the second output signal having a common characteristic with the first output signal.

10. The method of claim 9, wherein the first device comprises a first medical device or the second device comprises a second medical device.

11. The method of claim 9 or 10, wherein generating, by the first device, the instruction or the alert comprises:
determining that a physiological parameter is outside of a first range;
determining that a treatment parameter is outside of a second range;
determining that a charge level of a battery of the first device is below a threshold; or
determining whether the first device is communicatively coupled with a third device.

12. The method of claim 11, wherein generating, by the first device, the instruction or the alert comprises:
determining, that a subject has a condition comprising spontaneous circulation, lack of spontaneous circulation, spontaneous respiration, lack of spontaneous respiration, ineffectiveness of a paralytic, a cardiac arrhythmia, or resolution of a cardiac arrhythmia, and
generating the instruction to indicate a treatment or an alteration of a treatment that addresses the condition.

13. The method of claim 11 or 12, wherein the instruction is to administer a treatment to a subject, the treatment comprising assisted ventilation, administration of an electrotherapy, administration of a medication, or administration of chest compressions.

14. The method of claim 13, wherein:
the instruction is to administer the treatment at a particular time;
the first device outputs the first output signal at the particular time; and
the second device outputs the second output signal at the particular time.

15. The method of any one of claims 9-14, wherein the common characteristic comprises a common timing, a common wavelength, a common pulse pattern, or a common intensity.
